# EUROPEAN PATENT SPECIFICATION

(11) **EP 2 928 311 B1**
(45) Date of publication and mention of the grant of the patent: **17.08.2016**
(21) Application number: 13817890.0
(22) Date of filing: 29.11.2013
(51) Int. Cl.: A23C 9/12, A23C 19/024, C12M 1/26

(54) **PROCESS FOR DIRECT INOCULATION FROM FROZEN CONCENTRATED FERMENTS AND ASSOCIATED DEVICE**
VERFAHREN ZUR DIREKTEN INOKULATION AUS GEFRORENEN KONZENTRIERTEN FERMENTEN UND ZUGEHÖRIGE VORRICHTUNG
PROCÉDÉ D'INOCULATION DIRECTE À PARTIR DE FERMENTS CONCENTRÉS CONGELÉS, ET DISPOSITIF ASSOCIÉ

(30) Priority: 04.12.2012 FR 1261614
(43) Date of publication of application: 14.10.2015
(73) Proprietor: Chr. Hansen A/S, 2970 Hoersholm (DK)
(72) Inventor: POIGNAND, Jean-Paul, F-25000 Besancon (FR); LANCIAUX, Pascal, F-89100 Gron (FR); PIQUET, Marion, F-75014 Paris (FR); LANGEVIN, Emilien, F-91740 Pussay (FR); DIDELOT, Gilles, F-53240 Saint Germain Le Fouilloux (FR); ODINOT, Jean-Marie, F-54110 Remereville (FR); FAIVELEY, Marc, F-33140 Villenave d'Ornon (FR)
(86) International application number: PCT/EP2013/075057
(87) International publication number: WO 2014/086671

(56) References cited:
- WO-A1-2005/104861
- WO-A1-2011/134952
- FR-A1- 2 873 384
- MARUEJOULS A ET AL: "LES CULTURES CONCENTREES CONGELEES//Frozen concentrated cultures", TECHNIQUE LAITIERE, PARIS, FR, no. 976, May 1983 (1983-05), XP000669479, ISSN: 0040-1242

## Description

The present invention relates to a device and a process for continuous inoculation from frozen concentrated ferments requiring neither incubation, preculture or activation which have a potential health risk, nor interruption of the inoculation process during production.

Inoculation in the food-processing industry and in the dairy industry in particular is of essential importance for producing a product. Indeed, the industrial and qualitative performance levels of the final products depend on the nature and the efficiency of the ferments used and on their method of addition.

The obtaining of precultures, also known as starter cultures, i.e. prior to activation of the culture in order to reduce the lag phase, for the inoculation of milk is known from documents WO 200170935 and EP688864. Patent application WO 99/09838 describes a method for preparing a fresh product in which the starter culture can be in frozen form. These reactivation and/or dilution systems have the drawback of making it necessary to handle the concentrated ferments upstream of the inoculation phase, thereby risking contaminations.

Moreover, the fermentation of liquid medium to be inoculated with frozen concentrated ferments means that the manufacturer using them has to work in a batchwise mode for the inoculation and fermentation phases. Indeed, since the form and type of packaging is generally as bags or tins, the microorganisms must necessarily be added directly to the fermentation tank.

Other systems using frozen concentrated ferments, such as known from FR2873384, require the presence of a container for intermediate thawing of the ferments, which increases the risk of contaminations. The applicant has discovered, surprisingly, that the introduction of frozen concentrated ferments can be carried out by direct inoculation. This allows continuous inoculation without having to interrupt the fermentation process for the production of the final product. It thus becomes possible to substantially increase fermented product production rates.

The subject of the invention is thus a process for continuous inoculation of a food product, in particular a dairy product, with frozen concentrated ferments.

According to one general characteristic, the process comprises the following steps:
- frozen concentrated ferments are thawed by means of a microwave device or a water bath thawing device operating on a container containing frozen concentrated ferments,
- the thawed concentrated ferments are continuously injected, from the container, into a flow of liquid to be inoculated.

The subject of the invention is also an equipment for continuous inoculation of ferments into a liquid to be inoculated, wherein the ferments originate from frozen concentrated ferments, said equipment comprising a chamber for thawing a container comprising frozen concentrated ferments, said chamber comprising a microwave device or a water bath thawing device, an inoculation chamber provided with support means for installing at least two containers of thawed ferments and with at least one weighing device capable of continuously determining the remaining volume in the container being emptied, the equipment further comprising an injection circuit connecting the containers to a circuit for continuous feeding of the liquid to be inoculated, the injection circuit comprising a valve allowing switch from one container to another container and means for regulating the flow rate of the ferments in liquid form.

In one embodiment, the container containing the frozen concentrated ferments is stored at a temperature of -20 to -70°C prior to the thawing thereof.

Advantageously, once placed in the inoculation chamber, the container containing the thawed concentrated ferments is continuously weighed in order to determine, during emptying, the remaining volume of liquid ferments in the container weighed.

The injection of the thawed ferments is carried out via means of connection to a circuit for continuous feeding of liquid to be inoculated. These connection means may be pipes of an injection circuit, which can be cleaned and sterilized after each passage of the liquid to be inoculated in the line, or more or less flexible tubing provided with means of temporary connection, for example via clip-fastening or snap-fastening.

Microbial contamination of surfaces constitutes a danger to health through the possible contamination of foods during transformation thereof. This is, for example, the case when bacterial spores occur in biofilms, i.e. multicellular communities of microorganisms adhering to one another and to a surface. Indeed, bacterial spores exhibit remarkable resistance characteristics and contaminate the surfaces of the equipment and of connecting piping. For industrial manufacturers, the removal of biofilms in most cases requires the use of excessive hygiene procedures in order to ensure good preservation of the transformed foods, and to avoid food contaminations.

Thus, alternatively, the means of connection to a circuit for continuous feeding of liquid to be inoculated are disposable in order to ensure perfect sterility and easy use. These means of connection may also be changed according to the ferments used.

Preferably, the container after thawing is placed in an inoculation chamber at a pressure above atmospheric pressure.

Thus, in one embodiment of the invention, the inoculation chamber containing the thawed concentrated ferments is pressurized by means of a neutral sterile gas in order to maintain as far as possible in said chamber a constant pressure which thus facilitates the accuracy of the flow of the concentrated ferments. Furthermore, an overpressure in the thawing container limits the possibilities of contamination by outside air. An overpressure typically of 100 g/cm2 allows a more even metering.

In one embodiment of the invention, several containers are placed in a parallel arrangement in the inoculation chamber so that, when one of them is in the process of being emptied, at least one other container containing thawed concentrated ferments is on standby.

Preferably, by means of this process, a metered amount of thawed concentrated ferments is continuously introduced into a flow of liquid to be inoculated. This inoculated liquid will then be put in a fermentor, a tank for producing fermented products or a fermentation device, directly in the container intended to be marketed. In the case of a dairy product, for example, the fermentation unit may be a pot of dairy product.

This continuous inoculation has the effect of improving the regularity of the quality of the final products. The invention thus allows direct use, from their container, of the frozen concentrated ferments directly in the line of liquid to be inoculated without involving a risky intermediate phase. Any intermediate handling phase indeed inevitably leads to risks of accidental contamination which are detrimental to the whole of the subsequent process for producing the fermented product. Furthermore, directly inoculating into the line of liquid just before renneting makes it possible to limit any proliferation of phages and the creation of biofilms on the maturation zone.

Preferably, means for regulating the flow rate of the ferments in liquid form are placed upstream of the circuit for continuous feeding of the liquid to be inoculated. These means may be a pump.

The thawing time for these frozen concentrated ferments in the container is variable depending on the amounts of products present in the container.

Usually, for a microwave device, the thawing time for the frozen concentrated ferments is from 10 to 60 minutes.

The thawing time for the frozen concentrated ferments using a water bath thawing device is from 15 to 300 minutes.

In order to ensure a quick melting of the concentrated ferments without creating any large thermal shock which would be detrimental to the correct course of the subsequent steps of the production process, the temperature in the thawing chamber is regulated.

Preferably, the temperature of the ambient atmosphere in the microwave device of the thawing chamber is from 20 to 30°C and preferably 25°C.

Preferably, the temperature of the water bath in the water bath thawing device is from 15 to 45°C.

Preferably, the frozen concentrated ferments are stirred during the thawing in order to homogenize them and to avoid incompletely melted aggregates.

For this purpose, in one embodiment of the equipment, the thawing chamber may comprise means for stirring the container that are capable of distributing the heat evenly during the thawing.

Once placed in the inoculation chamber, the thawed liquid ferments are maintained at a relatively low temperature which may be from 2 to 12°C, or any other temperature compatible with maintaining the functionalities of the ferments. This makes it possible to limit as much as possible the resumption of the bacterial metabolism and to guarantee a quality of inoculation which is constant over time.

In one embodiment of the equipment, the inoculation chamber may comprise refrigeration means and means for maintaining the pressure above atmospheric pressure.

The inoculation chamber of the equipment may advantageously comprise means of homogenization of at least one container during emptying.

Thus, homogenization of the mixture of thawed and melted concentrated ferments during emptying makes it possible to ensure the homogeneity of the mixture of bacterial cultures constituting the ferments.

Preferably, the homogenization step comprises blending.

The frozen concentrated ferments can be packaged and stored in packaging with a more or less large capacity ranging from 200 g to several kilos. The transfer must be carried out under strict hygiene conditions in order to avoid any contamination detrimental to the whole of the subsequent fermentation process.

The frozen concentrated ferments used are composed of bacteria which are used for producing cheeses such as, for example, soft cheeses, cooked pressed cheeses, uncooked pressed cheeses, spun-curd cheeses, and fermented milks such as, for example, stirred or set, flavored or natural yoghurts, drinking yoghurts, sour cream and fromages frais and also for producing other fermented products such as, for example, wine.

The bacteria used may be mesophilic microorganisms, the optimum growth temperature of which is from 25 to 35°C. Among the mesophilic microorganisms typically used, mention may in particular be made of, for example, Lactococcus lactis subsp. lactis, Lactococcus lactis subsp. cremoris, Leuconostoc cremoris, Lactoccus lactis biovar. diacetylactis, Lactobacillus casei, Streptococcus durans, Streptococcus faecalis.

Use may also be made of thermophilic microorganisms, i.e. organisms of which the growth temperature may be from 35 to 45°C. Mention may in particular be made of, for example, Streptococcus thermophilus, Lactobacillus lactis, Lactobacillus helveticus, Lactobacillus delbrueckii subsp. bulgaricus and Lactobacillus acidophilus or any other appropriate microorganism.

Likewise, strictly anaerobic microorganisms of the bifidobacteria type, including Bifidus bifidum and Bifidobacterium longum (animalis) can be used.

Use may also be made of propionic bacteria such as Lactobacillus helveticus, Propionibacterium freudenreichii, Propionibacterium freudenreichii subsp. shermanii, etc.

The bacteria used may be wine bacteria, for example Oenococcus oeni (Leuconostoc oenos), Lactobacillus plantarum or Pedicoccus sp.

Use may also be made of yeasts of the family Saccharomycetaceae or molds such as Penicillium or Geotrichum.

The level of frozen concentrated ferment or concentrated bacterial culture inoculation varies according to the technologies and the products under consideration. Generally, this proportion is from 0.005% to 0.025% based on the total weight of the medium to be inoculated.

Generally, upon being produced, the ferments are frozen using liquid nitrogen, then stored at a temperature from -20 to -70°C.

Depending on their freezing temperature, the ferments can be stored for some time before use: up to 1 month in case of storing at -20°C, up to 6 months in case of storing at -40°C, and up to 12 months in case of storing at -45°C. Other purposes, characteristics and advantages will appear upon reading the following description of an embodiment and of a mode of implementation of the invention, given only as nonlimiting examples, and given with reference to the attached drawings in which:
- Figure 1 illustrates schematically a flowchart of the various steps of a process according to one mode of implementation of the invention,
- Figure 2 illustrates schematically a first embodiment according to the invention,
- Figure 3 illustrates schematically a second embodiment according to the invention,
- Figure 4 represents monitoring curves for the acidification of the culture medium at 43°C of the YF-L901 culture after thawing in a microwave oven, as a function of the temperature, -20°C or -40°C, for storage in frozen form,
- Figure 5 represents monitoring curves for the acidification of the culture medium at 30°C of the CHN-19 culture after thawing in a microwave oven, as a function of the temperature, -20°C or -40°C, for storage in frozen form,
- Figure 6 represents monitoring curves for the acidification of the culture medium at 30°C of the Flora Tradi 01 culture after thawing in a microwave oven, as a function of the temperature, -20°C or -40°C, for storage in frozen form,
- Figure 7 represents monitoring curves for the acidification of the culture medium at 40°C of the SCC-100 culture after thawing in a microwave oven, as a function of the temperature, -20°C or -40°C, for storage in frozen form,
- Figures 8a and 8b represent monitoring curves for the acidification of the culture medium (8a) and for the acidification rate (8b) at 30°C of the FMD-0046 culture after water bath thawing of frozen ferments stored at -45°C,
- Figures 9a and 9b represent monitoring curves for the acidification of the culture medium (9a) and for the acidification rate (9b) at 30°C of the R604 culture after water bath thawing of frozen ferments stored at -45°C,
- Figures 10a and 10b represent monitoring curves for the acidification of the culture medium (10a) and for the acidification rate (10b) at 40°C of the ssc1 culture after water bath thawing of frozen ferments stored at -45°C,
- Figures 11a and 11b represent monitoring curves for the acidification of the culture medium (11a) and for the acidification rate (11b) at 44°C of the YF-L703 culture after water bath thawing of frozen ferments stored at -45°C.

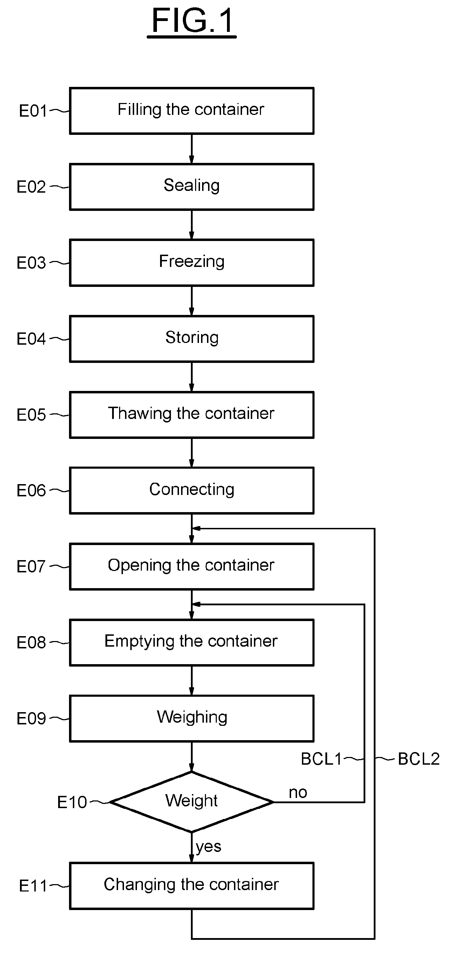

Represented schematically in Figure 1 is a flowchart of the various steps of an inoculation process according to one embodiment of the invention.

Prior to the inoculation, concentrated ferments are frozen in containers.

For this, in a first step E01, a container is sterilely filled with concentrated ferments. The containers may be packaging of more or less large capacity ranging from 200 g to several kilograms that are capable of maintaining concentrated ferments composed of bacteria that are used for producing cheeses, fermented milks and other fermented products.

Then, in a step E02, the orifice of the container is sealed, still while maintaining sterility, so as to obtain a hermetically sealed container filled with concentrated ferments.

In a subsequent step E03, the ferments are frozen and then, in a step E04, these frozen ferments are stored at a temperature from -20 to -70°C for a relatively long time of a few days to several months.

It is possible to repeat steps E01 to E04 with different containers so as to obtain a plurality of containers comprising the same frozen concentrated ferments.

For the inoculation, in step E05, frozen ferments are thawed in situ in one of the containers previously kept frozen. This thawing step is carried out via microwave means or a water bath thawing device acting on the container and more particularly on the frozen ferments contained in the container. The microwave or water bath thawing means may comprise a thawing chamber into which the container is introduced, the thawing chamber being maintained at an ambient temperature of about 25°C in order to ensure rapid melting of the concentrated ferments without creating a large detrimental heat shock. In the embodiment presented, the frozen concentrated ferments are stirred during the thawing, in order to distribute the heat evenly and to avoid incompletely melted aggregates.

In a subsequent step E06, the container which has undergone thawing is connected to a disposable injection circuit.

In a subsequent step E07, the container connected to the injection circuit is installed in an inoculation chamber and the container is opened.

The thawed container is then emptied in a step E08. During the emptying, the inoculation chamber is pressurized with a neutral sterile gas in order to maintain a constant pressure therein as much as possible and thus to facilitate the accuracy of the flow of the concentrated ferments. The thawed liquid ferments are also maintained at a temperature from 2 to 12°C, so as to limit as much as possible the resumption of the bacterial metabolism and to guarantee an inoculation quality which is constant over time.

While being emptied, the container is regularly weighed, in a step E09, so as to determine the amount of ferments remaining in the container.

Next, in a step E10, the weight measured in the preceding step is compared to a threshold value corresponding to the weight of the empty or almost empty container. In addition, depending on the weight of the container, and therefore depending on the amount of ferments remaining in said container, the container-emptying operation is continued by resuming it in step E08 via a loop BCL1, or the virtually empty container is exchanged with a full thawed container in a step E11. The thawing of the full container may have been initiated during the emptying of the previous container, or before the beginning of the emptying of said previous container, for example after the beginning of the thawing of said previous container using another thawing chamber.

These steps of emptying a container, weighing, and optionally changing container according to the volume of remaining ferments are carried out via a loop BCL2.

The parallel arranging of several containers in an inoculation chamber and step E011 of exchanging a container to be emptied make it possible to obtain a continuous inoculation process wherein a metered amount of thawed concentrated ferments is continuously introduced into a flow of liquid to be inoculated, wherein the inoculated liquid can then be introduced in a fermentor, a tank for producing fermented products or a device for fermentation, directly in the container intended to be marketed.

This continuous inoculation results in improving the regularity of the quality of the final products.

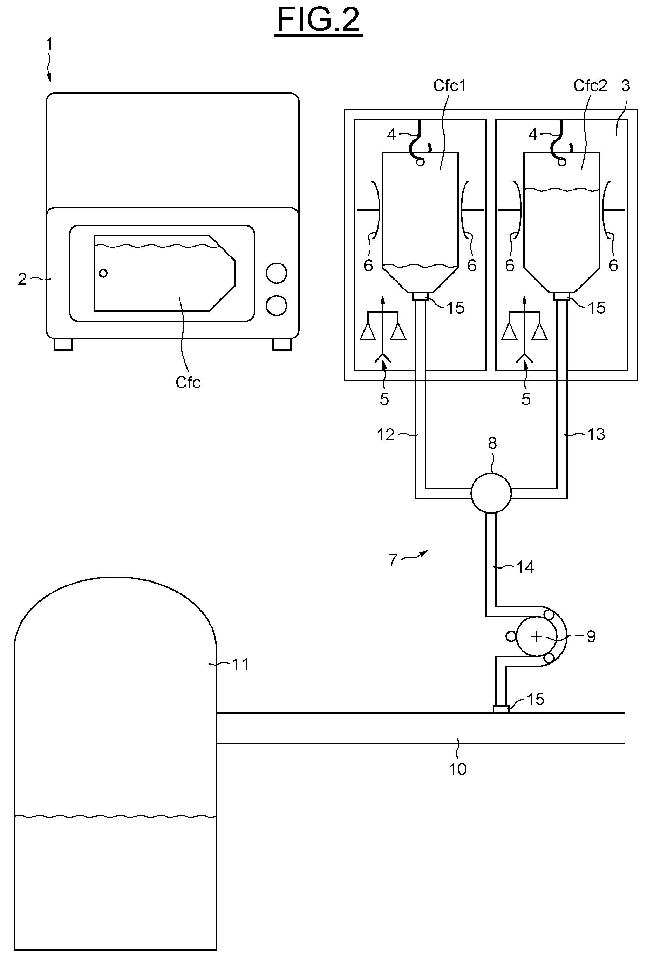

Represented schematically in Figure 2 is an inoculation equipment 1 according to a first embodiment of the invention.

The equipment 1 comprises a thawing chamber 2 comprising a microwave device or any other high-frequency machine capable of thawing a container of frozen concentrated ferments Cfc according to step E05 of the process illustrated in Figure 1. The thawing chamber 2 comprises means for stirring the ferments during the thawing, which are not represented in the figure, for homogenization of the ferments.

The equipment 1 also comprises an inoculation chamber 3. The inoculation chamber illustrated in this figure comprises two support means 4 each capable of supporting a container of thawed concentrated ferments Cfc1 and Cfc2, for example a vertical attachment device or a device for gripping the container, comprising a set of plates for holding the container in place and/or a hook. It is possible to store certain types of concentrated ferments once thawed in the inoculation chamber 3 for several hours and up to 24 hours, but preferably between 4 and 8 hours without particular effect on the resumption of the bacterial metabolism or on the activity of the bacteria constituting the concentrated ferments.

The inoculation chamber 3 of the equipment 1 comprises, moreover, means 5 for weighing the container in order to deduce the volume of the remaining ferments during emptying (steps E08 to E10). The inoculation chamber 3 also comprises homogenization means 6 for homogenizing the ferments located in the container. By way of nonlimiting example, use may be made of a plurality of plates applying a different pressure per plate which varies with passing time. The homogenization can be carried out continuously or intermittently as required.

In addition, the inoculation chamber 3 may comprise air-conditioning means not represented in Figure 2. Thus, the inoculation chamber 3 can be refrigerated at a temperature of from 2 to 12°C throughout the duration of the inoculation.

The inoculation chamber 3 may comprise a plurality of means for supporting the container of thawed concentrated ferments Cfc, the Cfc containers being connected via an injection circuit 7 to a circuit for continuous feeding 10 of the liquid to be inoculated. In the embodiment illustrated in Figure 2, the injection circuit 7 comprises a valve 8 connected to a first container Cfc1 via a first circuit portion 12, to a second container Cfc2 via a second circuit portion 13 and to the feeding circuit 10 via a third circuit portion 14. The valve 8 thus makes it possible to change container Cfc1 or Cfc2 without interrupting the injection process.

The injection circuit 7 also comprises a pump 9 installed on the third circuit portion 14, consequently downstream of the valve 8. The pump 9 serves to regulate the flow rate of afferent liquid concentrated ferments of the container Cfc1 or Cfc2 in place in the inoculation chamber 3. The regulating pumps used, such as pump 9, can be proportioned according to the flow rate of the main circuit of the medium inoculated; typically in the dairy industry, the pump flow rates range from 0.1 I/hour to 4 I/hour, for equipment of 2 to 10000 I/hour, up to 0.75 I/hour to 12 I/hour for equipment of 15000 to 30000 l/hour.

The injection circuit 7 may also comprise connecting means 15 at the level of the container(s) Cfc1 and Cfc2 in the inoculation chamber 3, and at the level of the junction between the circuit portion 14 and the feeding circuit 10.

These connecting means 15 make it possible to sterilize and clean the injection circuit 7 more easily. In another embodiment, these connecting means make it possible to change the portions 12, 13 and 14 of the injection circuit 7 in order to replace them with others which are sterile, during, for example, the changing in the composition of the ferments being used to inoculate the pipe 10 for feeding of the liquid to be inoculated.

The inoculation chamber 3 may also comprise means, not represented in the figure, for checking the pressure inside the inoculation chamber 3.

The equipment 1 also comprises a fermentation unit 11 connected to the circuit 10 for feeding the liquid to be inoculated. The inoculation of said liquid is carried out by means of a tapping on the pipe of the feeding circuit 10, making it possible to connect the third circuit portion 14 of the injection circuit 7.

The fermentation unit 11 is in this case reproduced in the form of a fermentor. Of course, it is also possible to envisage that the fermentation unit 11 is a tank for producing fermented products or a device for fermentation directly in the container intended to be marketed, for example a pot of dairy product.

The quantification of the thawed ferments is an essential part of the fermentation unit inoculation process.

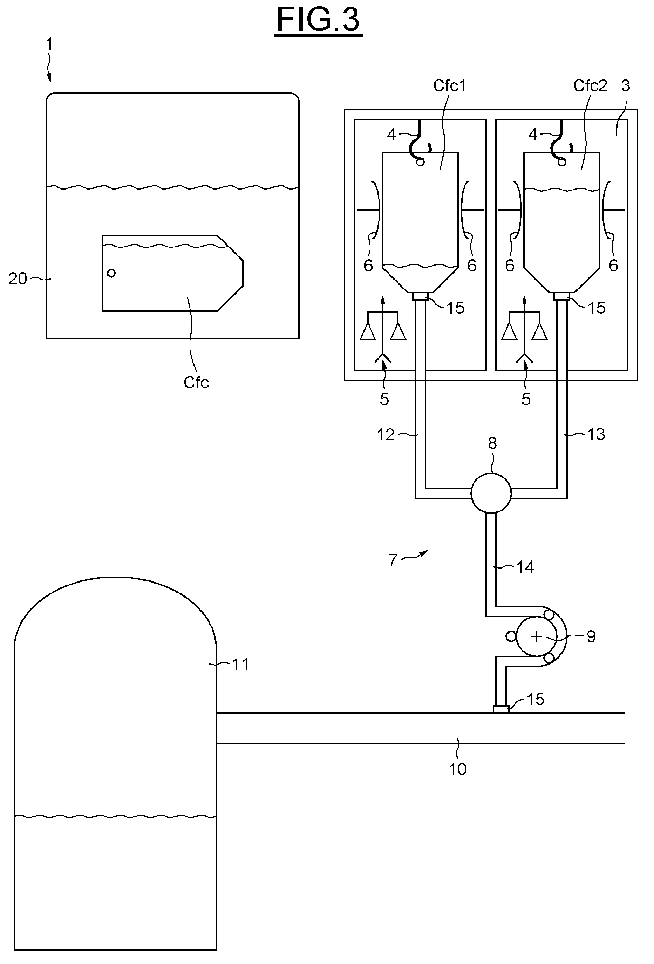

Figure 3 schematically shows inoculation equipment 1 according to a second embodiment of the invention. Same parts as in Figure 2 are assigned the same reference numbers.

In the second embodiment shown in Figure 3, equipment 1 comprises a thawing chamber 20 comprising a water bath thawing device instead of a microwave device.

Whatever the embodiment of the invention, the inoculation equipment makes it possible to obtain a continuous and accurate on line flow of a small amount of concentrated ferments from frozen concentrated ferments for inoculating a fermentation unit. The invention thus allows the use of the frozen concentrated ferments directly from their container, directly in the line of liquid to be inoculated without a risky intermediate phase being involved. Any intermediate handling phase, in fact, inevitably leads to risks of accidental contamination which are detrimental to the whole of the subsequent process for producing the fermented product. Furthermore, directly inoculating into the line of liquid just before renneting makes it possible to limit any possible phage proliferation.

### Example 1: Monitoring the acidification of the culture medium following thawing using a microwave device

The ferments YF-L901 (composed of Streptococcus thermophilus and of Lactobacillus bulgaricus), CHN-19, Flora Tradi 01 (multistrain ferments composed of Lactococcus lactis subspecies lactis, subspecies cremoris and subspecies biovar diacetylactis and of Leuconostoc cremoris) and SSC-100 (Streptococcus thermophilus) are packaged in sterile pouches of 5 liters, i.e. 2.5 kg of ferments in a form of frozen granules stored at a temperature of either- 40°C or -20°C.

The pouches are placed in a microwave oven set on 600 W (Sairem, France).

The ferments previously stored at -40°C were subjected to microwaves for 30 minutes to achieve complete melting. The ferments previously stored at -20°C required 25 minutes for complete melting.

The pouches are placed on a stirrer throughout the thawing in order to ensure homogeneous melting of the concentrated ferments.

The tests for acidification of the culture medium were carried out on milk reconstituted at 9.5% solids content from skimmed milk powder, heated at 99°C for 30 min. The inoculation dose is 0.02% for YF-L901 with a maturation temperature of 43°C, 0.01% for SSC-100 with a maturation temperature of 40°C and 0.01% with CHN-19 and Flora Tradi 01 with a maturation temperature of 30°C.

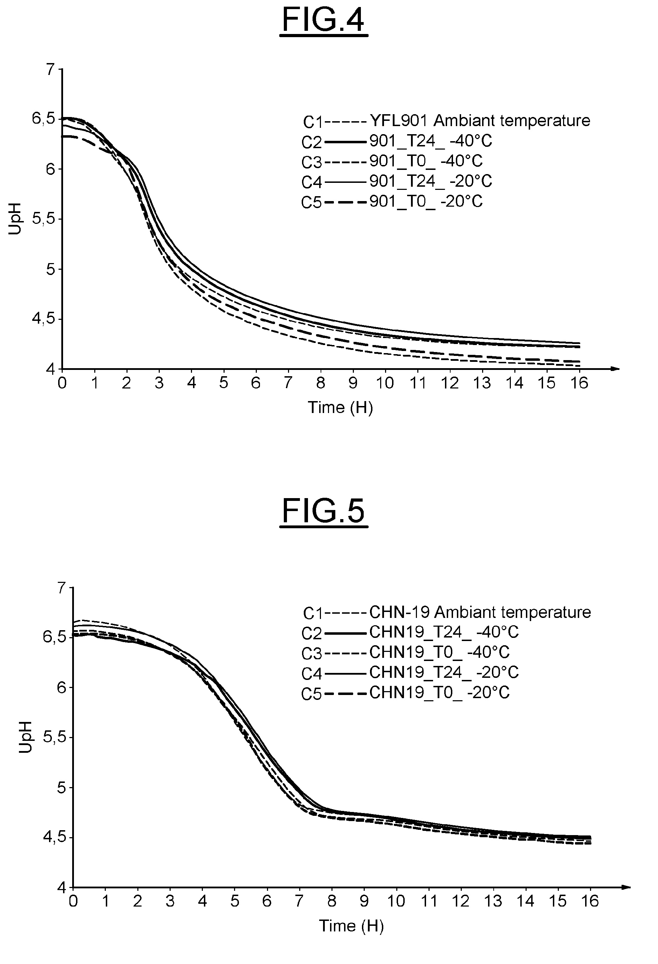

The results of the monitoring of the acidifying activity of each of the strains tested are given below as curves of variation in pH of the inoculated medium as a function of time, the test strains having been previously thawed in a microwave device (Figures 4 to 7).

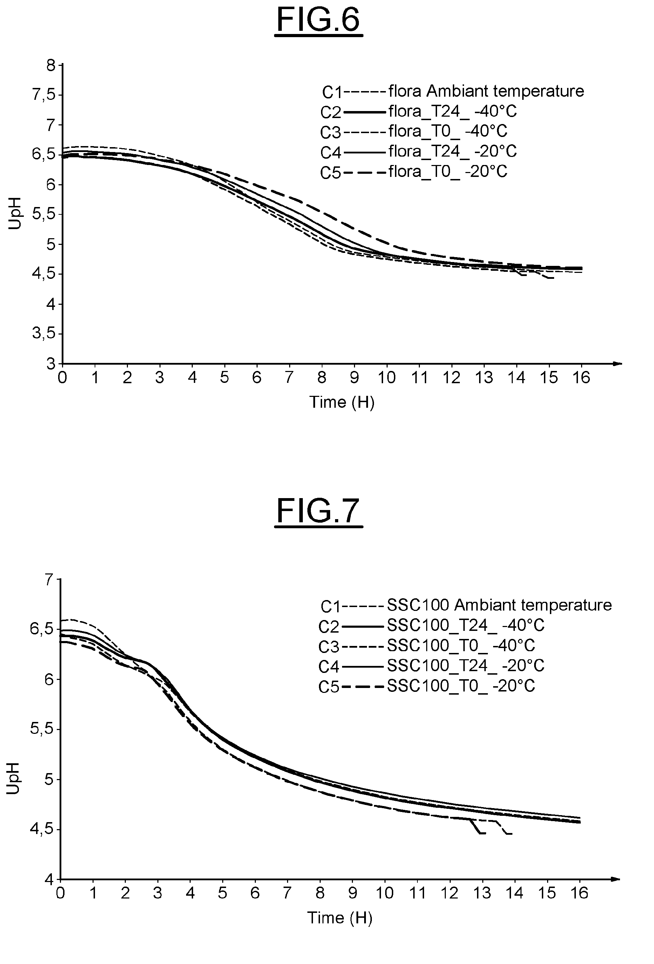

In particular, Figure 4 represents monitoring curves for the acidification of the culture medium of the YF-L901 culture, Figure 5 represents monitoring curves for the acidification of the culture medium of the CHN-19 culture, Figure 6 represents monitoring curves for the acidification of the culture medium of the Flora Tradi 01 culture at 30°C, and Figure 7 represents monitoring curves for the acidification of the culture medium of the SCC-100 culture, after thawing in a microwave oven, as a function of the temperature for storage in frozen form.

In each of the figures, the first curve referenced C1 corresponds to the control for culture of the ferments without previous thawing, curves C3 and C5 represent the curves obtained just after thawing for containers stored before thawing respectively at-40°C and -20°C. Curves C2 and C4 represent the curves obtained after thawing followed by storing at 4°C for 24 h, for containers stored before thawing respectively at -40°C and -20°C.

The acidification monitorings for the various strains tested allow one to deduce that there is no significant effect of the storage temperature of the ferments before thawing on the acidifying activity performance levels.

Thus, it was demonstrated that it is possible to store various types of concentrated bacterial cultures for several days at a temperature of -20 to -40°C, and then to thaw them in a microwave oven without particular effect on the resumption of the bacterial metabolism and on the activity, in particular acidifying activity, of the ferments under consideration.

### Example 2: Monitoring the acidification of the culture medium following thawing using a water bath

Various bacterial cultures were thawed using a water bath the temperature of which is regulated according to each bacterium.

These bacteria are the following ones:
- FMD-0046 comprised of a mixture of Lactococcus lactis subspecies lactis and Lactococcus lactis subspecies cremoris
- R604 comprised of a mixture of Lactococcus lactis subspecies lactis, Lactococcus lactis subspecies cremoris and Lactococcus lactis subspecies lactis biovar diactetylactis
- SSC-1 comprised of a mixture of Streptococcus thermophilus
- YF-L703 comprised of a mixture of Streptococcus thermophilus and of Lactobacillus delbruckeii subspecies bulgaricus

For mesophilic ferments such as FMD-0046 and R-604, the water bath temperature is 30°C for a thawing duration of 45 minutes. For thermophilic ferments such as SSC-1 and YF-L703, the water bath temperature is 40°C for a thawing duration of 30 minutes.

The ferment container is stirred throughout the thawing duration in order to ensure homogeneous melting and to avoid incompletely melted lumps.

The tests for acidification of the culture medium were carried out on milk reconstituted at 9.5% dry solid content from skimmed milk powder, heated at 99°C for 30 min. The inoculation dose is 0.01% for FMD-0046 with a maturation temperature of 30°C, 0.01% for R-604 with a maturation temperature of 30°C, 0.01% for SSC-1 with a maturation temperature of 40°C, and 0.02% for YF-L703 with a maturation temperature of 44°C.

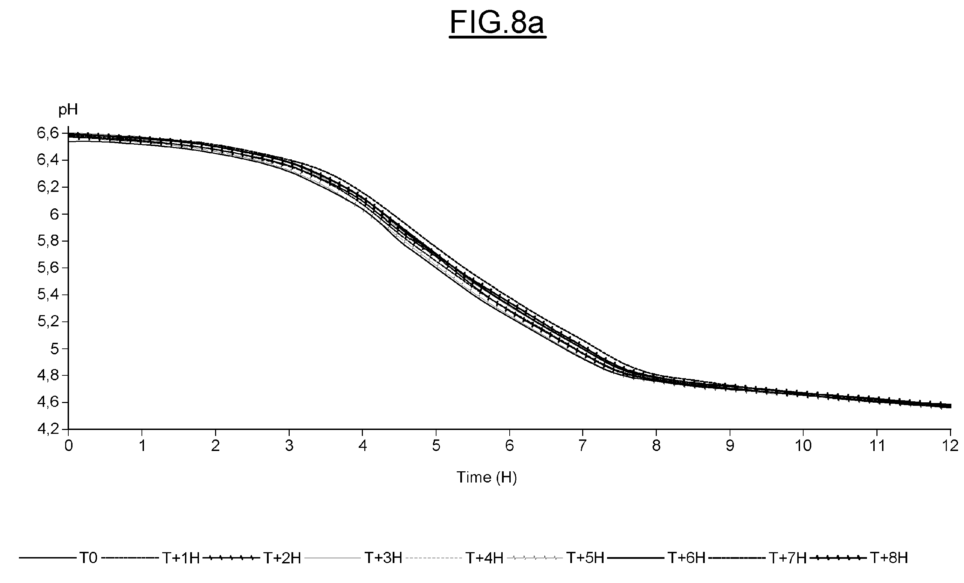

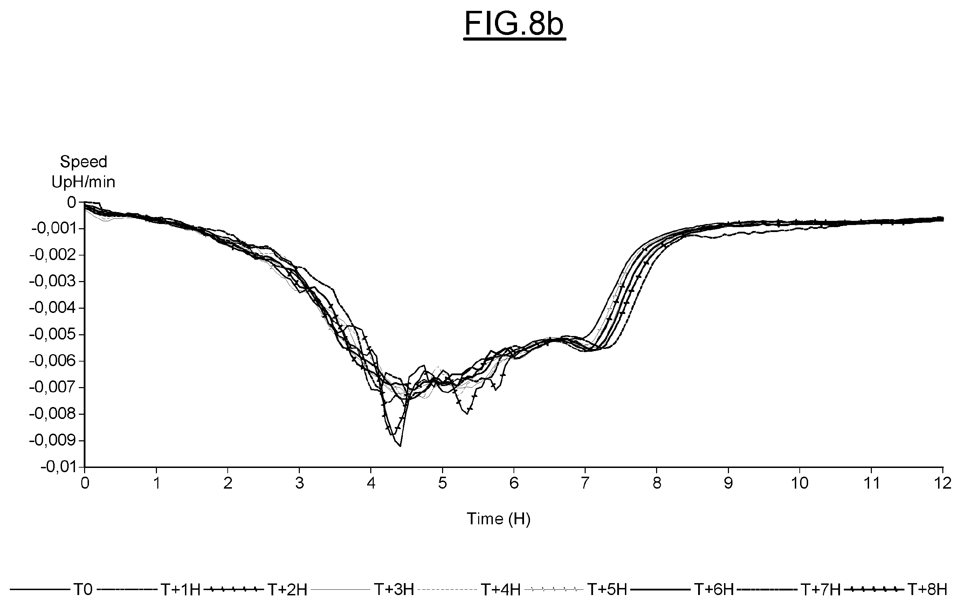

The results of the monitoring of the acidifying activity of each of the strains tested are given below as curves of variation in pH of the inoculated medium as a function of time, the test strains having been previously thawed in the water bath device following storage at -45°C (Figures 8 to 11).

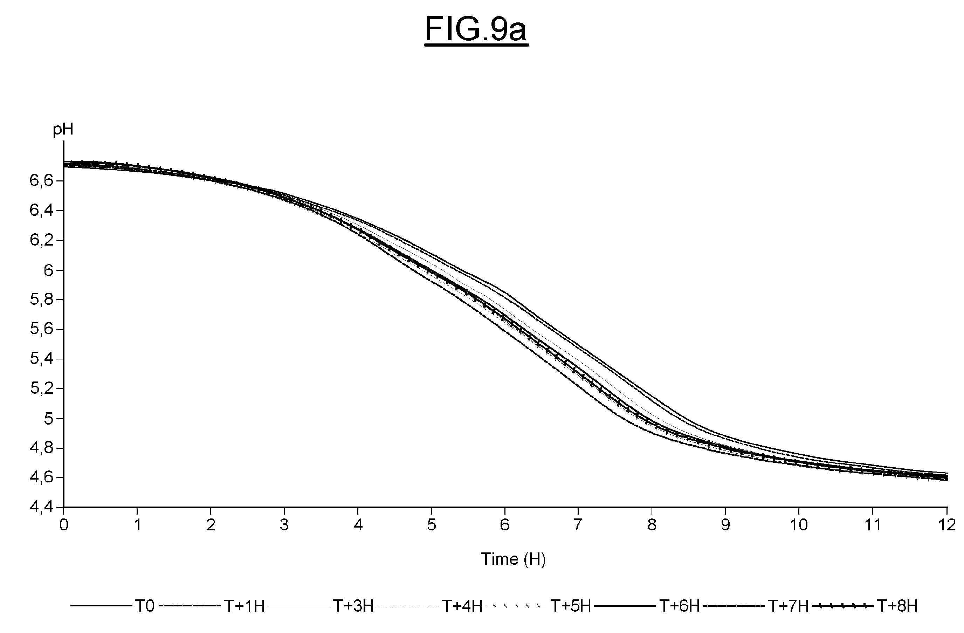

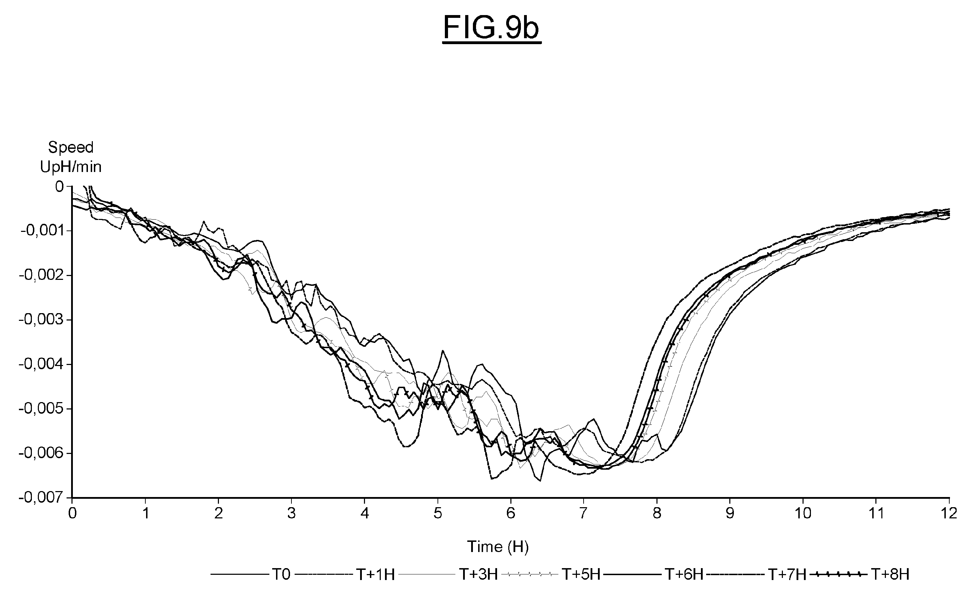

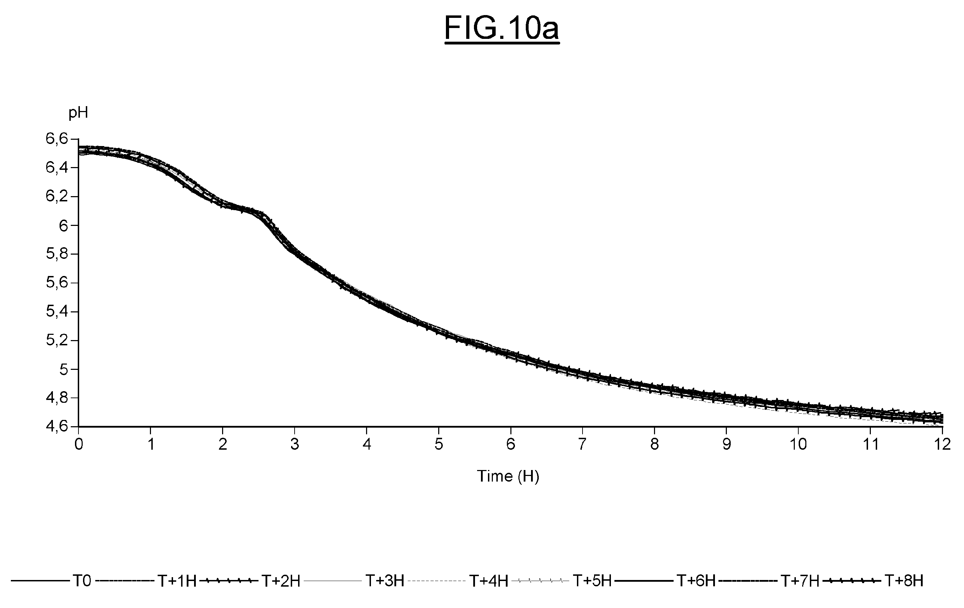

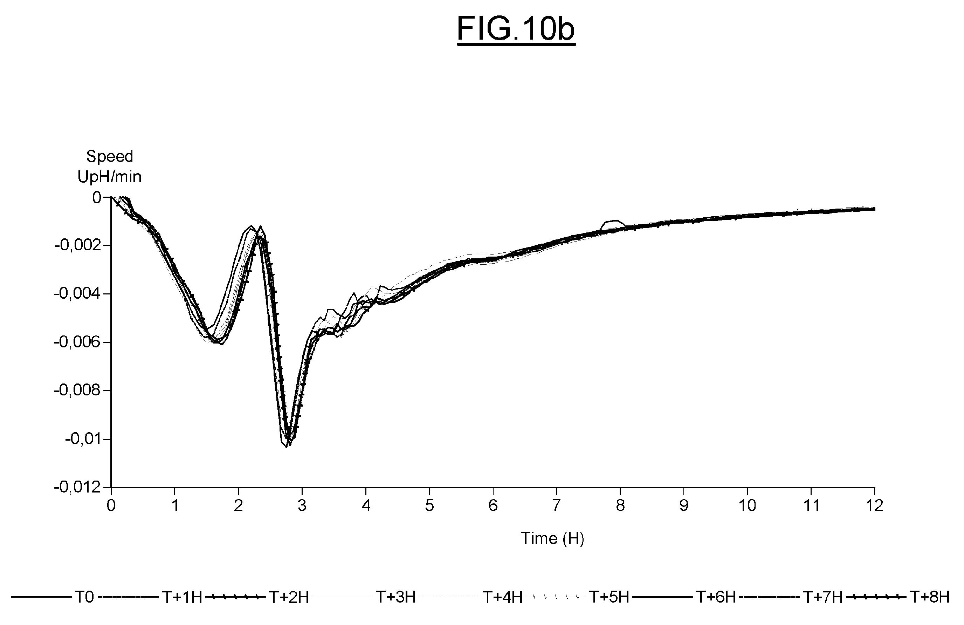

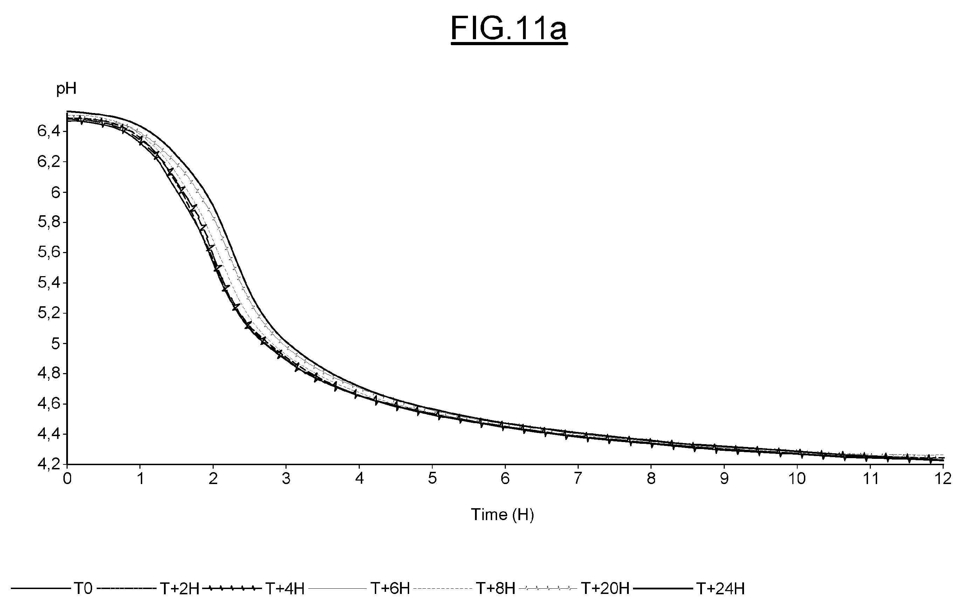

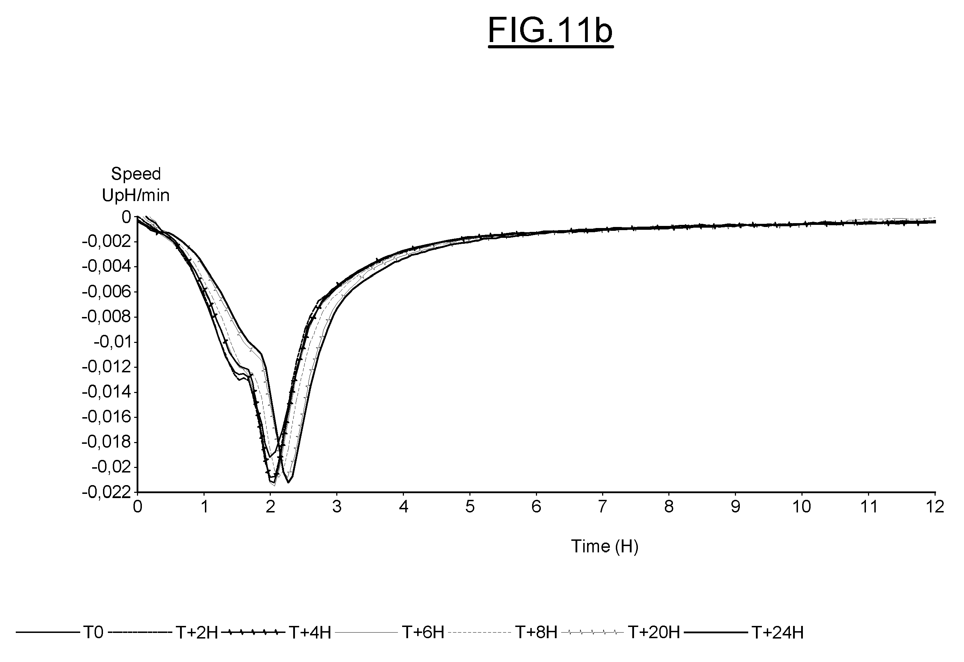

In particular, Figures 8a and 8b represent monitoring curves for the acidification of the culture medium of the FMD-0046 culture, Figures 9a and 9b represent monitoring curves for the acidification of the culture medium of the R-604 culture, Figures 10a and 10b represent monitoring curves for the acidification of the culture medium of the SSC-1 culture, and Figures 11a and 11b represent monitoring curves for the acidification of the culture medium of the YF-L703 culture at 44°C, after thawing of the frozen ferments in a water bath.

The acidification monitorings for the various strains tested allow one to deduce that there is no significant effect of the duration of low temperature storage of the ferments after thawing on the acidifying activity performance levels, in the operating conditions used.

## Claims

1. Process for continuous inoculation of a food product, in particular a dairy product, with ferments, **characterized by** the following steps:
- frozen concentrated ferments are thawed by means of a microwave device or a water bath thawing device acting on a container containing frozen concentrated ferments (E05),
- the thawed concentrated ferments are continuously injected, from the container, into a flow of liquid to be inoculated (E07, E08).

2. Process for continuous inoculation of a food product with ferments according to claim 1, wherein the container containing the frozen concentrated ferments is stored at a temperature of from -20 to -70°C prior to thawing thereof (E03).

3. Process for inoculation according to any one of claims 1 and 2, wherein the container is continuously weighed in order to determine, during emptying, the remaining volume in the container weighed (E09, E10).

4. Process for inoculation according to any one of claims 1 to 3, wherein the injection is carried out via means of connection, which are changed according to the ferments used.

5. Process for inoculation according to any one of claims 1 to 4, wherein the container is placed in an inoculation chamber at a pressure above atmospheric pressure.

6. Process for inoculation according to any one of claims 1 to 5, wherein several containers are placed in parallel arrangement, one of them being emptied while at least one other is on standby.

7. Process for inoculation according to any one of claims 1 to 6, wherein the flow rate of the ferments injected in liquid form is regulated.

8. Process for inoculation according to any one of claims 1 to 7, wherein the thawing time of the frozen concentrated ferments using the water bath thawing device is from 15 to 300 minutes.

9. Process for inoculation according to any one of claims 1 to 8, wherein the water bath temperature in the water bath thawing device is from 15°C to 45°C.

10. Process for inoculation according to any one of claims 1 to 7, wherein the thawing time of the frozen concentrated ferments using the microwave device is from 10 to 60 minutes.

11. Process for inoculation according to any one of claims 1 to 10, wherein the frozen concentrated ferments are stirred during the thawing.

12. Process for inoculation according to any one of claims 1 to 11, wherein the thawed liquid ferments are maintained at a temperature ranging from 2 to 12°C.

13. Process for inoculation according to any one of claims 1 to 12, wherein the thawed liquid ferments are homogenized during emptying.

14. Process for inoculating according to any one of claims 1 to 13, wherein the homogenization comprises blending.

15. Equipment (1) for continuous inoculation of ferments into a liquid to be inoculated, the ferments originating from frozen concentrated ferments, comprising a chamber (2, 20) for thawing a container comprising frozen concentrated ferments (Cfc), said chamber comprising a microwave device or a water bath thawing device, an inoculation chamber (3) provided with support means (4) for installing at least two containers of thawed ferments (Cfc1 and Cfc2) and with at least one weighing device (5) capable of continuously determining the remaining volume in the container being emptied, the equipment (1) also comprising an injection circuit (7) connecting the containers (Cfc1 and Cfc2) to a circuit (10) for continuous feeding of the liquid to be inoculated, the injection circuit (7) comprising a valve (8) enabling the switching from one container (Cfc1) to another container (Cfc2) and means (9) for regulating the flow rate of the ferments in liquid form.

16. Equipment according to claim 15, wherein said thawing chamber (2) comprises means for stirring the container (Cfc) that are capable of evenly distributing the heat during the thawing.

17. Equipment according to any one of claims 15 and 16, wherein said inoculation chamber (3) comprises refrigeration means and means for maintaining the pressure above atmospheric pressure.

18. Equipment according to any one of claims 15 to 17, wherein said inoculation chamber (3) comprises means (6) for homogenization of at least one container.

## Patentansprüche

1. Verfahren zur kontinuierlichen Inokulation eines Lebensmittels, insbesondere eines Milchprodukts, mit Fermenten, **gekennzeichnet durch** die folgenden Phasen:
- gefrorene konzentrierte Fermente werden mittels einer Mikrowellenvorrichtung oder einer Wasserbadauftauvorrichtung aufgetaut, die auf einen Behälter einwirkt, der gefrorene konzentrierte Fermente (E05) enthält,
- die aufgetauten konzentrierten Fermente werden fortlaufend aus dem Behälter in einen Strom von zu inokulierender Flüssigkeit (E07, E08) eingespritzt.

2. Verfahren zur kontinuierlichen Inokulation eines Lebensmittels mit Fermenten nach Anspruch 1, wobei der Behälter, der die gefrorenen konzentrierten Fermente enthält, bei einer Temperatur von -20 bis -70°C gelagert wird, bevor man sie auftaut (E03).

3. Verfahren zur Inokulation nach einem beliebigen der Ansprüche 1 und 2, wobei der Behälter fortlaufend gewogen wird, um während der Entleerung das verbleibende Volumen in dem gewogenen Behälter (E09, E10) zu bestimmen.

4. Verfahren zur Inokulation nach einem beliebigen der Ansprüche 1 bis 3, wobei die Einspritzung mittels Verbindungsmitteln durchgeführt wird, die je nach verwendeten Fermenten gewechselt werden.

5. Verfahren zur Inokulation nach einem beliebigen der Ansprüche 1 bis 4, wobei der Behälter in einer Inokulationskammer bei einem Druck über dem atmosphärischen Druck gelagert wird.

6. Verfahren zur Inokulation nach einem beliebigen der Ansprüche 1 bis 5, wobei mehrere Behälter parallel angeordnet sind und einer von ihnen entleert wird, während mindestens ein anderer auf Standby ist.

7. Verfahren zur Inokulation nach einem beliebigen der Ansprüche 1 bis 6, wobei die Durchflussmenge der Fermente, die in flüssiger Form eingespritzt werden, geregelt wird.

8. Verfahren zur Inokulation nach einem beliebigen der Ansprüche 1 bis 7, wobei die Auftauzeit der gefrorenen konzentrierten Fermente unter Verwendung der Wasserbad-Auftauvorrichtung 15 bis 300 Minuten beträgt.

9. Verfahren zur Inokulation nach einem beliebigen der Ansprüche 1 bis 8, wobei die Wasserbadtemperatur in der Wasserbadauftauvorrichtung 15°C bis 45°C beträgt.

10. Verfahren zur Inokulation nach einem beliebigen der Ansprüche 1 bis 7, wobei die Auftauzeit der gefrorenen konzentrierten Fermente unter Verwendung der Mikrowellenvorrichtung 10 bis 60 Minuten beträgt.

11. Verfahren zur Inokulation nach einem beliebigen der Ansprüche 1 bis 10, wobei die gefrorenen konzentrierten Fermente während des Auftauens umgerührt werden.

12. Verfahren zur Inokulation nach einem beliebigen der Ansprüche 1 bis 11, wobei die aufgetauten flüssigen Fermente bei einer Temperatur von 2 bis 12°C gehalten werden.

13. Verfahren zur Inokulation nach einem beliebigen der Ansprüche 1 bis 12, wobei die aufgetauten flüssigen Fermente während der Entleerung homogenisiert werden.

14. Verfahren zum Inokulieren nach einem beliebigen der Ansprüche 1 bis 13, wobei die Homogenisierung die Mischung umfasst.

15. Ausrüstung (1) für die kontinuierliche Inokulation von Fermenten in eine zu inokulierende Flüssigkeit, wobei die Fermente aus gefrorenen konzentrierten Fermenten stammen, mit einer Kammer (2, 20), um einen Behälter mit gefrorenen konzentrierten Fermenten (Cfc) aufzutauen, wobei die Kammer eine Mikrowellenvorrichtung oder eine Wasserbadauftauvorrichtung umfasst, eine Inokulationskammer (3) mit Träger (4) zum Installieren mindestens zweier Behälter von aufgetauten Fermenten (Cfc1 und Cfc2) und mindestens eine Wiegevorrichtung (5) zur kontinuierlichen Bestimmung des verbleibenden Volumens in dem Behälter, der gerade entleert wird, wobei die Ausrüstung (1) auch einen Einspritzkreislauf (7) umfasst, der die Behälter (Cfc1 und Cfc2) mit einem Kreislauf (10) zur kontinuierlichen Zuführung der zu inokulierenden Flüssigkeit verbindend, wobei der Einspritzkreislauf (7) ein Ventil (8) umfasst, das den Wechsel von einem Behälter (Cfc1) zu einem anderen Behälter (Cfc2) ermöglicht, sowie Mittel (9), um die Durchflussmenge der Fermente in flüssiger Form zu regulieren.

16. Ausrüstung nach Anspruch 15, wobei besagte Auftaukammer (2) Mittel zum Umrühren des Behälters (Cfc) umfasst, die während des Auftauens die Hitze gleichmäßig verteilen.

17. Ausrüstung nach einem beliebigen der Ansprüche 15 und 16, wobei die Inokulationskammer (3) Kühlmittel umfasst und Mittel zum Beibehalten des Druckes über dem atmosphärischen Druck.

18. Ausrüstung nach einem beliebigen der Ansprüche 15 bis 17, wobei die Inokulationskammer (3) Mittel (6) zur Homogenisierung mindestens eines Behälters umfasst.

## Revendications

1. Procédé d'inoculation continue d'un produit alimentaire, en particulier un produit laitier, avec des ferments, **caractérisé par** les étapes suivantes:
- des ferments concentrés congelés sont décongelés au moyen d'un dispositif à micro-ondes ou d'un dispositif de décongélation à bain d'eau agissant sur un récipient contenant des ferments concentrés congelés (E05),
- les ferments concentrés décongelés sont injectés de façon continue, depuis le récipient, dans un flux de liquide à inoculer (E07, E08).

2. Procédé d'inoculation continue d'un produit alimentaire avec des ferments selon la revendication 1, où le récipient contenant les ferments concentrés congelés est stocké à une température comprise entre -20 et -70°C avant leur décongélation (E03).

3. Procédé d'inoculation selon l'une quelconque des revendications 1 et 2, où le récipient est pesé de façon continue afin de déterminer, pendant le vidage, le volume restant dans le récipient pesé (E09, E10).

4. Procédé d'inoculation selon l'une quelconque des revendications 1 à 3, où l'injection est effectuée grâce à des moyens de connexion, qui sont changés selon les ferments utilisés.

5. Procédé d'inoculation selon l'une quelconque des revendications 1 à 4, où le récipient est placé dans une chambre d'inoculation à une pression supérieure à la pression atmosphérique.

6. Procédé d'inoculation selon l'une quelconque des revendications 1 à 5, où plusieurs récipients sont placés dans un agencement parallèle, l'un d'entre eux étant vidé tandis qu'au moins un autre est en mode d'attente.

7. Procédé d'inoculation selon l'une quelconque des revendications 1 à 6, où le débit des ferments injectés sous forme liquide est réglé.

8. Procédé d'inoculation selon l'une quelconque des revendications 1 à 7, où le temps de décongélation des ferments concentrés congelés utilisant le dispositif de décongélation à bain d'eau est de 15 à 300 minutes.

9. Procédé d'inoculation selon l'une quelconque des revendications 1 à 8, où la température de bain d'eau dans le dispositif de décongélation à bain d'eau est comprise entre 15°C et 45°C.

10. Procédé d'inoculation selon l'une quelconque des revendications 1 à 7, où le temps de décongélation des ferments concentrés congelés utilisant le dispositif à micro-ondes est de 10 à 60 minutes.

11. Procédé d'inoculation selon l'une quelconque des revendications 1 à 10, où les ferments concentrés congelés sont agités pendant la décongélation.

12. Procédé d'inoculation selon l'une quelconque des revendications 1 à 11, où les ferments liquides décongelés sont maintenus à une température qui varie de 2 à 12°C.

13. Procédé d'inoculation selon l'une quelconque des revendications 1 à 12, où les ferments liquides décongelés sont homogénéisés pendant le vidage.

14. Procédé d'inoculation selon l'une quelconque des revendications 1 à 13, où l'homogénéisation comprend un mélange.

15. Équipement (1) pour l'inoculation continue de ferments dans un liquide à inoculer, les ferments provenant de ferments concentrés congelés, comprenant une chambre (2, 20) pour la décongélation d'un récipient comprenant des ferments concentrés congelés (Cfc), ladite chambre comprenant un dispositif à micro-ondes ou un dispositif de décongélation à bain d'eau, une chambre d'inoculation (3) pourvue de moyens de support (4) pour installer aux moins deux récipients de ferments décongelés (Cfc1 et Cfc2) et avec au moins un dispositif de pesage (5) capable de déterminer de façon continue le volume qui reste dans le récipient en train d'être vidé, l'équipement (1) également comprenant un circuit d'injection (7) reliant les récipients (Cfc1 et Cfc2) à un circuit (10) pour l'alimentation continue du liquide à inoculer, le circuit d'injection (7) comprenant une soupape (8) permettant de changer d'un récipient (Cfc1) à un autre récipient (Cfc2) et des moyens (9) pour régler le débit des ferments sous forme liquide.

16. Équipement selon la revendication 15, où ladite chambre de décongélation (2) comprend des moyens pour agiter le récipient (Cfc) qui sont capables de distribuer uniformément la chaleur pendant la décongélation.

17. Équipement selon l'une quelconque des revendications 15 et 16, où ladite chambre d'inoculation (3) comprend des moyens de réfrigération et des moyens pour maintenir la pression au-dessus de la pression atmosphérique.

18. Équipement selon l'une quelconque des revendications 15 à 17, où ladite chambre d'inoculation (3) comprend des moyens (6) pour l'homogénéisation d'au moins un récipient.
